Europäisches Patentamt

European Patent Office

Office européen des brevets

(19)

(11) Numéro de publication : **0 128 115**
**B1**

(12) **FASCICULE DE BREVET EUROPÉEN**

(45) Date de publication du fascicule du brevet :
04.03.87

(51) Int. Cl.⁴ : **A 61 F 5/01**

(21) Numéro de dépôt : **84810166.3**

(22) Date de dépôt : **04.04.84**

(54) Appareil orthopédique.

(30) Priorité : **30.05.83 CH 2953/83**

(43) Date de publication de la demande :
**12.12.84 Bulletin 84/50**

(45) Mention de la délivrance du brevet :
**04.03.87 Bulletin 87/10**

(84) Etats contractants désignés :
**CH DE FR GB IT LI NL**

(56) Documents cités :
**US-A- 1 904 942**
**US-A- 2 844 143**
**US-A- 3 086 522**
**US-A- 3 345 654**

(73) Titulaire : **Paratte, Bernard**
**23, Général-Dufour**
**CH-2300 La-Chaux-de-Fonds (CH)**

(72) Inventeur : **Paratte, Bernard**
**23, Général-Dufour**
**CH-2300 La-Chaux-de-Fonds (CH)**

(74) Mandataire : **Steiner, Martin et al**
**c/o AMMANN INGENIEURS-CONSEILS EN PRO-
PRIETE INTELLECTUELLE SA BERNE Schwarztorstrasse 31**
**CH-3001 Bern (CH)**

EP 0 128 115 B1

## Description

La présente invention concerne un appareil orthopédique comprenant au moins un support orthopédique ayant une surface d'appui de forme au moins approximativement sphérique, le support orthopédique comprenant une semelle permettant l'appui de la plante des pieds et dont le prolongement évite aux orteils de supporter le poids des draps et couvertures et au moins une bride prévue pour la fixation du support orthopédique au pied.

Un tel appareil est connu du brevet US-A-3 345 654. Cependant, cet appareil ne permet aucune orientation du pied du patient et il ne comporte aucun moyen pour supporter le mollet.

On connaît aussi du brevet US-A-3 892 231 un dispositif de correction de l'inclinaison ou des déformations des os, particulièrement pour les enfants. Le dispositif est en forme de soulier en deux parties, la partie avant étant réglable en orientation relativement à la partie arrière dans un plan sagittal et dans un plan perpendiculaire à celui-ci. Cependant, un tel dispositif ne comporte aucun moyen pour supporter le mollet et il n'est pas prévu pour maintenir dans une position donnée le pied ou la jambe d'un patient couché, pour soulager ce patient.

On connaît encore du brevet US-A-3 086 522 un appareil orthopédique pour corriger des déformations des os, plus particulièrement destiné à corriger des déformations du pied ou de la jambe chez les enfants. Il comporte un certain nombre de réglages tels que celui permettant de donner à la partie avant du pied une position déterminée par rapport au talon, celui permettant l'inclinaison du plan du pied par rapport à l'axe de la jambe, celui permettant l'inclinaison de la direction de l'axe de la cuisse par rapport à l'axe de la partie de la jambe au-dessous du genou. Cependant, l'appareil ne permet pas au patient d'étendre la jambe et la tige support empêche la position couchée du patient. Un réglage en hauteur du pied n'est pas possible. L'appareil n'est pas conçu pour soulager la douleur après une opération par une position adéquate, réglable de la jambe en position couchée du patient.

En conséquence, le but de la présente invention est de réaliser un appareil orthopédique permettant de supporter le mollet et de maintenir le pied ou la jambe d'un patient couché dans une position réglable donnée et désirée par le médecin pour soulager le patient avant ou après une opération du pied, de la jambe, du genou ou de la hanche. L'appareil doit en outre éviter le poids des draps et couvertures sur les orteils et dégager le talon pour que celui-ci n'appuie pas.

Pour atteindre ce but, l'appareil orthopédique selon l'invention est réalisé comme décrit dans les revendications.

La gouttière, fixée de manière amovible au support orthopédique, soutient le mollet du patient et elle peut s'adapter au support orthopédique, avec n'importe quelle orientation dans un domaine angulaire solide déterminé. La base comporte des réglages permettant l'inclinaison du support orthopédique dans le plan de la semelle et dans le plan sagittal, de sorte que la position du pied peut être ajustée selon les indications du médecin, la coulisse permettant cependant au support orthopédique de rester au niveau de la base quelle que soit l'inclinaison de la semelle par rapport à cette dernière. Le prolongement de la semelle permet d'éviter le poids des draps et couvertures sur les orteils et le revêtement amovible à l'intérieur du support orthopédique est prévu de manière à laisser le talon sans contact avec l'appareil.

L'invention va être décrite ci-après, à titre d'exemple et à l'aide du dessin dans lequel :

La figure 1 est une vue latérale de l'appareil selon l'invention,

La figure 2 est une vue en plan de l'appareil de la figure 1,

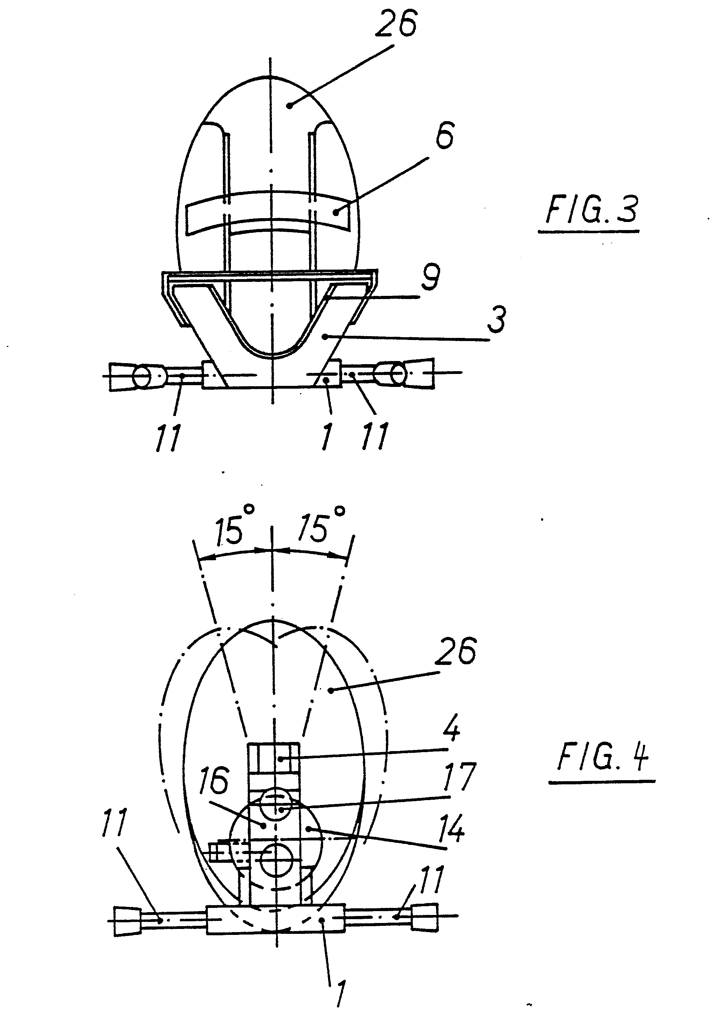

La figure 3 est une vue arrière de l'appareil,

La figure 4 est une vue avant de l'appareil, et

Les figures 5, 6, 7 et 8 sont respectivement des vues arrière, latérale, avant et en plan de la base.

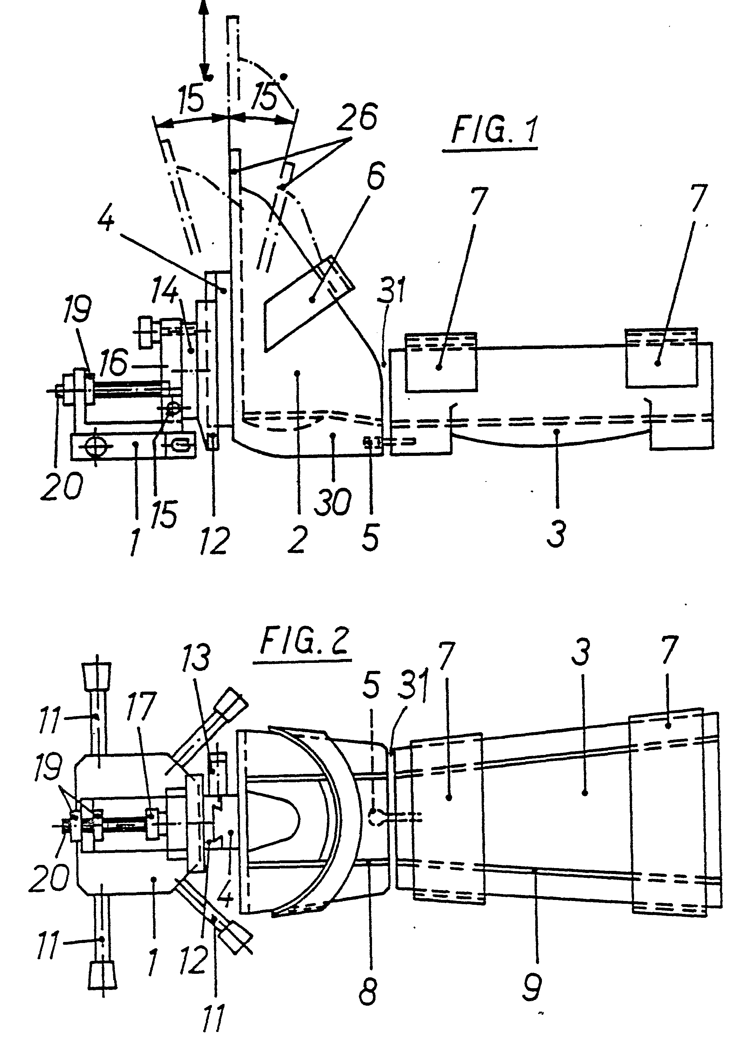

Les figures 1 et 2 montrent respectivement une vue latérale et une vue en plan de l'appareil selon l'invention. Celui-ci comprend une base 1 montée par une coulisse 12 sur une partie correspondante 4 fixée à la semelle 26 du support orthopédique 2 décrit ci-dessus. Une vis 13 permet de bloquer la coulisse dans la position désirée. Une gouttière 3 destinée à soutenir le mollet est fixée de manière amovible dans la base 30 du support orthopédique 2 à l'aide d'une demi-rotule 5. La gouttière est en forme de cylindre ou de cône tronqué ouvert vers le haut pour recevoir la jambe. La surface intérieure de la gouttière est aussi recouverte d'un revêtement auto-adhésif 9 similaire au revêtement 8 du support orthopédique 2. Des brides d'attache 7 auto-adhésives permettent de fixer la gouttière à la jambe du patient. Les figures 1 et 2 montrent qu'il existe un jeu 31 entre la gouttière 3 et le support orthopédique 2. Ce jeu et le montage sur rotule 5 permettent à la gouttière de s'adapter à n'importe quelle orientation du support orthopédique 2 dans un domaine angulaire solide déterminé tout en restant horizontale, la gouttière pouvant aussi tourner autour de son axe longitudinal.

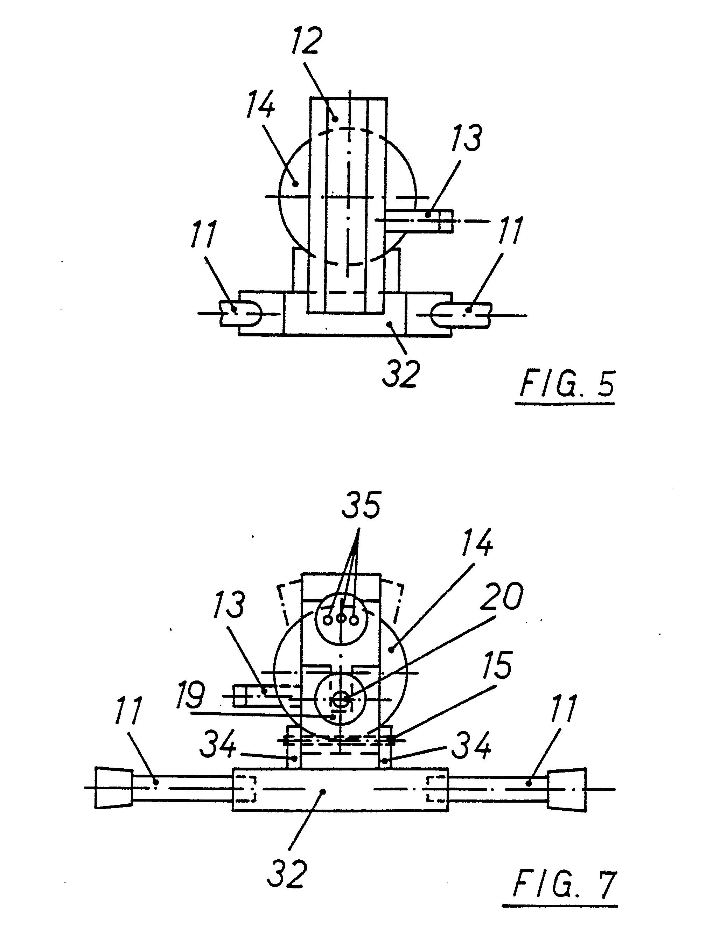

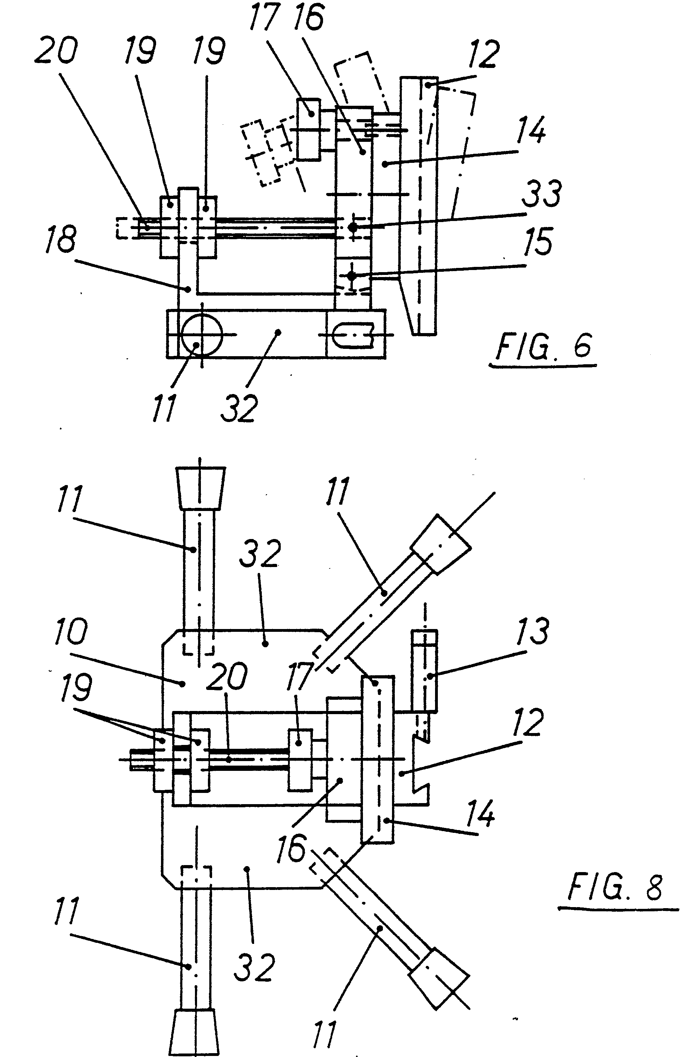

La base 1 est illustrée dans les figures 5 à 8. Elle se compose d'une plaque de base 32 en bois ou en plastique à laquelle sont fixés quatre bras 11 comme indiqué en figure 8, ces bras donnant une bonne stabilité à l'appareil. Sur la plaque de base 32 est montée une équerre 18 dont la partie verticale supporte dans une fente médiane une vis 20 avec écrou et contre-écrou 19, l'autre extrémité de la vis 20 étant insérée dans une plaque 16 et tenue dans cette dernière par un axe 33 autour duquel elle pivote. La plaque 16 est montée pivotante autour d'un axe 15 fixé dans deux montants 34 solidaires de la partie horizontale de l'équerre 18. Une plaque circulaire 14 est fixée rigidement à la coulisse 12 et elle peut tourner autour de son axe monté dans la plaque 16. La plaque circulaire 14 comporte à sa partie supérieure trois trous borgnes 35 indiqués schématiquement en figure 8 et dans lesquels l'extrémité en forme de goupille d'un plot 17 peut s'engager. Le plot 17 est toujours tiré en direction de la coulisse par un ressort (non représenté) disposé dans l'épaisseur de la plaque 16.

Le mécanisme décrit ci-dessus permet différentes orientations spatiales de la coulisse 12, et en conséquence du support orthopédique 2 monté sur cette coulisse. Lorsque le plot 17 est engagé dans l'ouverture 35 centrale de la plaque circulaire 14, la semelle 26 du support orthopédique est dans la position médiane indiquée en trait plein en figure 4. En retirant le plot 17 contre la force du ressort non représenté, la plaque circulaire 14 est libérée et il est possible de lui faire exécuter une rotation autour de son axe, par exemple mais non exclusivement de 15° vers la gauche ou vers la droite, et de la verrouiller dans chacune de ces positions extrêmes en relachant le plot 17 dont l'extrémité s'engage alors dans le trou borgne 35 correspondant, verrouillant ainsi la plaque circulaire 14 et la coulisse dans cette position extrême. En conséquence, la semelle 26 se trouve dans l'une ou l'autre des positions extrêmes représentées en trait-points en figure 4, ces positions étant respectivement décalées angulairement, dans l'exemple décrit, de 15° par rapport à la position médiane. Ce qui précède montre que le support orthopédique peut être ajusté angulairement selon trois positions dans le plan de la semelle 26.

En desserrant l'écrou et le contre-écrou 19, l'extrémité de gauche de la vis 20 (figure 1 ou 6) est libérée, ce qui permet de faire basculer la plaque 16 avec la plaque circulaire 14, la coulisse 12 et le support orthopédique 2 autour de l'axe 15, vers l'avant ou vers l'arrière. Par rapport à la position médiane avec la semelle 26 en position verticale comme illustré en figure 1, il est possible de faire basculer le support orthopédique dans le plan sagittal par exemple mais pas exclusivement de 15° vers l'avant et vers l'arrière. Dans chaque cas, la coulisse 12 permet d'ajuster la hauteur du support orthopédique de manière que sa base 30 se trouve au niveau de la plaque 32.

Ce qui précède montre que le réglage du support orthopédique dans le plan sagittal est continu et réalisé par la vis 20 et les écrou et contre-écrou 19. Le réglage dans le plan de la semelle 26 est discontinu et il n'est prévu que trois positions d'indexage. Il est clair cependant que ce réglage pourrait être rendu continu par exemple à l'aide d'une vis sans fin et que le réglage dans le plan sagittal pourrait sans autre être à crans. De même la coulisse 12, 4 pourrait être remplacée par un système de tube et de piston sans sortir du cadre de l'invention. Les déplacements angulaires dans le plan de la semelle et le plan sagittal ne sont pas limités à +

ou — 15° mais ils peuvent être réalisés avec tout angle différent désiré.

Le support orthopédique 2 peut être utilisé seul, éventuellement mais pas obligatoirement avec la gouttière 3 dans le cas de phlébites ou de douleurs dans les jambes.

L'appareil orthopédique complet est utilisé par exemple dans le cas de ligaments déchirés, avant l'opération et après l'opération du pied, de la jambe, du genou et de la hanche. Dans le cas de paralysie flasque ou en cas d'atteinte cérébrale, le patient est inconscient et l'appareil selon l'invention est alors utilisé pour maintenir ses pieds en position correcte, afin de prévenir leur déformation permanente qui ne permettrait plus au patient, ultérieurement, de poser le talon sur le sol.

L'appareil selon l'invention peut être mis en location, tout comme les cannes et les béquilles. L'invention se propose en premier lieu de soulager les patients, de venir en aide aux chirurgiens et de simplifier le travail du personnel soignant.

**Revendications**

1. Appareil orthopédique pour un patient alité comprenant au moins un support orthopédique (2) ayant une surface d'appui (25) de forme au moins approximativement sphérique, le support orthopédique comprenant une semelle (26) permettant l'appui de la plante des pieds et dont le prolongement évite aux orteils de supporter le poids des draps et couvertures et au moins une bride (6) prévue pour la fixation du support orthopédique au pied, caractérisé en ce qu'il comprend en outre une gouttière (3) destinée à supporter le mollet et une base (1) qui repose sur le lit susceptible d'être couplée de manière amovible à la semelle (26) et comportant des moyens de réglage (17 ; 19, 20) permettant d'orienter relativement au lit la semelle (26) dans son plan et dans un plan sagittal.

2. Appareil selon la revendication 1, caractérisé en ce que les moyens de réglage permettant l'orientation de la semelle (26) dans son plan de la semelle (26) comprennent un élément (14) lié à la semelle et susceptible de pivoter autour d'un axe passant par son centre et perpendiculaire à la semelle ledit axe étant monté dans une plaque (16) solidaire de la base (1), ledit élément (14) étant indexé angulairement par un plot (17) monté dans la plaque (16), tiré par un ressort en direction du support orthopédique (2) et dont l'extrémité intérieure s'engage dans des trous (35) de la plaque circulaire (14).

3. Appareil selon la revendication 2, caractérisé en ce que ledit élément (14) est réglé angulairement de manière continue, par exemple par un engrenage à vis sans fin.

4. Appareil selon l'une des revendications 1 à 3, caractérisé en ce que les moyens de réglage de la semelle (26) dans le plan sagittal comprennent une plaque (16) liée à la semelle et supportée sur la base (1) de manière à pivoter dans le plan sagittal, des moyens de réglage (19, 20) de l'inclinaison de la plaque (16) étant prévus entre cette plaque et la base (1).

5. Appareil selon l'une des revendications 1 à 4, caractérisé en ce que la base (1) est liée à la semelle (26) par un élément coulissant (4, 12) permettant le réglage en hauteur du support orthopédique (2) quelle que soit son inclinaison dans le plan sagittal.

6. Appareil selon la revendication 1, caractérisé en ce que la gouttière (2) destinée à soutenir la jambe est en forme de cylindre ou de cône tronqué ouvert vers le haut et qu'elle comprend au moins une bride (7) pour la fixation à la jambe, la gouttière étant normalement couplée avec jeu par une demi-rotule (5) au support orthopédique (2) et pouvant aussi tourner autour de son axe longitudinal.

7. Appareil selon l'une des revendications 1 ou 6, caractérisé en ce que le support orthopédique (2) et la gouttière (3) comportent à l'intérieur des revêtements amovibles (8, 9) (par exemple peau de mouton, coussin de gel, etc.) pour permettre une hygiène complète, la partie du revêtement du support orthopédique dans la base (30) de celui-ci ne s'étendant pas jusqu'à la semelle (26) pour laisser le talon sans contact avec l'appareil.

8. Appareil selon l'une des revendications 1, 6 ou 7, caractérisé en ce que les revêtements amovibles (8, 9) et les brides (6, 7) sont auto-adhésifs pour faciliter et accélérer leur mise en place et leur remplacement.

## Claims

1. An orthopaedic device for a patient confined to bed, comprising at least one orthopaedic support (2) having an approximately spherical shaped resting surface (25), the orthopaedic support comprising a sole (26) for permitting the plantar surfaces to rest thereon, the prolongation of which permitting the toes to be free from supporting the weight of the clothes and blankets and at least a clamp (6) provided for the attachment of the orthopaedic support to the foot, characterized in that it further comprises a cradle-splint (3) provided for supporting the calf and a base (1) which rests on the bed and is capable to be removably coupled to the sole (26) and comprising adjusting means (17 ; 19, 20) permitting to orient the sole (26) in its plan and in a sagittal plane with respect to the bed.

2. A device according to claim 1, characterized in that the adjusting means permitting an orientation of the sole (26) in the plane thereof comprise an element (14) connected to the sole and capable to pivot about an axis passing through its center and being perpendicular to the sole, said axis being mounted in a plate (16) integral with the base (1), said element (14) being angularly indexed by a stud (17) mounted in the plate (16), pulled by a spring towards the orthopaedic support (2) and the internal end of which engaging in holes (35) of the circular plate (14).

3. A device according to claim 2, characterized in that said element (14) is capable to be angularly continuously adjusted by means of e. g. a worm gear.

4. A device according to one of the claims 1 to 3, characterized in that the adjusting means of the sole in the sagittal plane comprise a plate (16) connected to the sole and supported on the base (1) in such a way as to pivot in the sagittal plane, adjusting means of the inclination in the plate (16) being provided between said plate and the base (1).

5. A device according to one of the claims 1 to 4, characterized in that the base 1 is connected to the sole (26) by a sliding element (4, 12) permitting to adjust the height of the orthopaedic support (2) independently from the inclination thereof in the sagittal plane.

6. A device according to claim 1, characterized in that the cradle-splint (2) provided for supporting the leg is cylindrically shaped or in form of a truncated cone open at the top and in that it comprises at least one clamp (7) for its attachment to the leg, the cradle-splint being normally coupled with play to the orthopaedic support (2) by means of a half swivel (5) and being also capable to rotate about its longitudinal axis.

7. A device according to one of the claims 1 or 6, characterized in that the orthopaedic support (2) and the cradle-splint (3) comprise at the inside removable linings (8, 9) (e. g. sheepskin, gel cushion, etc.) for permitting a complete hygiene, the portion of the lining of the orthopaedic support lying in the base (30) thereof non extending until the sole (26) in order to leave the heel without contact with the device.

8. A device according to one of the claims 1, 6 or 7, characterized in that the removable linings (8, 9) and the clamps (6, 7) are self-adhesive in order to facilitate and accelerate their setting in place and their exchange.

## Patentansprüche

1. Orthopädisches Gerät für einen bettlägerigen Patienten, welches mindestens eine orthopädische Stütze (2) mit einer angenäherten sphärischen Auflagefläche aufweist, wobei die orthopädische Stütze eine Sohle (26) aufweist, welche die Auflage der Plantarfläche erlaubt und deren Verlängerung den Zehen das Tragen des Gewichtes der Tücher und Decken abnimmt, und mindestens ein Band (6), welches für die Halterung der orthopädischen Stütze am Fuss vorgesehen ist, aufweist, dadurch gekennzeichnet, dass es weiter eine Schiene (3), welche zur Auflage der Wade vorgesehen ist und eine Unterlage (1), welche auf dem Bett liegt und entfernbar mit der Sohle (26) gekuppelt werden kann und Einstellmittel (17 ; 19, 20) aufweist, welche die Sohle in ihrer Ebene und in einer Sagittalebene in Bezug auf das Bett einzustellen erlauben.

2. Gerät nach Anspruch 1, dadurch gekennzeichnet, dass die Einstellmittel, welche die Orientierung der Sohle (26) in ihrer Ebene erlauben, ein mit der Sohle gekuppeltes Element (14), welches um eine durch ihre Mitte und senkrecht zur Sohle verlaufende Achse drehen kann, aufweisen, und dass die Achse in einer mit der Unterlage vereinigten Platte (16) angebracht ist, wobei das Element (14) durch einen Zapfen winkelabhängig indexiert wird, welcher durch eine Feder in Richtung der orthopädischen Stütze (2) gezogen wird, wobei sein inneres Ende in Löcher (35) der kreisförmigen Platte (14) greift.

3. Gerät nach Anspruch 2, dadurch gekennzeichnet, dass das Element (14) winkelabhängig kontinuierlich, z. B. durch ein Schneckengetriebe, geregelt werden kann.

4. Gerät nach einem der Ansprüche 1 bis 3, dadurch gekennzeichnet, dass die Mittel zur Einstellung der Sohle (26) in der Sagittalebene eine mit der Sohle verbundene und auf der Unterlage (1) abgestützte Platte (16), welche in der Sagittalebene drehen kann, aufweisen, wobei Einstellmittel (19, 20) für die Neigung der Platte (16) zwischen dieser Platte und der Unterlage (1) vorgesehen sind.

5. Gerät nach einem der Ansprüche 1 bis 4, dadurch gekennzeichnet, dass die Unterlage (1) mit der Sohle (26) durch ein gleitbares Element (4, 12) verbunden ist, welches die Höheneinstellung der orthopädischen Stütze (2) unabhängig von ihrer Neigung in der Sagittalebene einzustellen erlaubt.

6. Gerät nach Anspruch 1, dadurch gekennzeichnet, dass die zur Abstützung des Beines vorgesehene Schiene (3) eine zylindrische oder kegelstumpfige, nach oben geöffneter Form besitzt, und dass sie mindestens ein Band (7) für die Halterung am Bein aufweist, wobei die Schiene normalerweise mit Spiel durch eine Halbkugel (5) mit der orthopädischen Stütze gekuppelt ist, so dass sie auch um ihre longitudinale Achse drehen kann.

7. Gerät nach einem der Ansprüche 1 oder 6, dadurch gekennzeichnet, dass die orthopädische Stütze (2) und die Schiene (3) entfernbare Ueberzüge (8, 9) (z. B. Schafleder, Gelkissen, usw.) aufweisen, um eine vollkommene Hygiene zu erlauben, wobei der Teil des Ueberzuges der orthopädischen Stütze, der sich in dieser befindet, sich nicht bis zur Sohle (26) erstreckt, um die Ferse ohne Kontakt mit dem Gerät zu lassen.

8. Gerät nach einem der Ansprüche 1, 6 oder 7, dadurch gekennzeichnet, dass die entfernbaren Ueberzüge (8, 9) und die Bänder (6, 7) selbstklebend sind, um ihren Einbau und ihren Wechsel zu erleichtern und zu beschleunigen.

0 128 115

FIG. 1

FIG. 2

FIG. 3

FIG. 4

FIG. 5

FIG. 7

3

*FIG. 6*

*FIG. 8*